Europäisches Patentamt

(19) European Patent Office

Office européen des brevets

(11) Publication number: **0 160 681**
**B1**

# (12) EUROPEAN PATENT SPECIFICATION

(45) Date of publication of patent specification: **02.03.88**

(21) Application number: **84904009.2**

(22) Date of filing: **18.10.84**

(86) International application number:
**PCT/SE84/00343**

(87) International publication number:
**WO 85/01743 25.04.85 Gazette 85/10**

(51) Int. Cl.⁴: **C 12 N 11/00, C 12 N 11/14, C 12 P 1/00**

(54) AEROBIC MICROBIOLOGICAL METHOD.

(30) Priority: **19.10.83 SE 8305729**

(43) Date of publication of application:
**13.11.85 Bulletin 85/46**

(45) Publication of the grant of the patent:
**02.03.88 Bulletin 88/09**

(84) Designated Contracting States:
**AT BE CH DE FR GB LI LU NL SE**

(56) References cited:
**DE-A-2 443 502**
**SE-A-75 020 578**
**US-A-4 165 281**

(73) Proprietor: **GULLFIBER AB**
**Box 501**
**S-260 50 Billesholm (SE)**

(72) Inventor: **CEDERBERG, Nils, Erik, Anders**
**Slöjdgatan 16**
**S-260 50 Billesholm (SE)**

(74) Representative: **Wiklund, Erik et al**
**AWAPATENT AB Box 5117**
**S-200 71 Malmö (SE)**

Courier Press, Leamington Spa, England.

## Description

The present invention relates to an aerobic microbiological method.

As used herein, the expression microbiological method refers to such methods as include microorganisms, such as bacteria, fungi, algae, cells and the like.

There are two types of microbiological methods, viz such as are carried out with the addition of oxygen (aerobic methods) and such as are carried out without the addition of oxygen (anaerobic methods). In methods of the former type, to which the invention belongs, a substrate solution and an oxygen-containing gas, such as air, oxygen-enriched air or pure oxygen, are supplied to a microorganism which then grows and multiplies itself and/or produces metabolites. The desired product in the method may be either the microorganism itself, as in microbial protein production, or a metabolite from the microorganism, such as penicillin in the production of penicillin, or the process implies removal of an undesired substance which was initially present in the substrate solution and which the microorganism had used for its growth.

Different aerobic processes are so notorious that an enumeration or detailed description thereof is superfluous here. The conditions for the microbiological method, such as the composition of the substrate solution, pressure, temperature etc. are established in a manner which is known per se for conventional microbiological methods. Thus, for instance, the method is normally carried out at ambient pressure, the temperature being about 0—100°C, usually 20—80°C, and preferably 20—40°C.

In order that the microbiological process should proceed as favourably as possible, it is of importance that the supply of oxygen-containing gas (for the sake of simplicity, referred to as air hereinbelow) and of substrate solution is efficient. Generally, one tries to achieve this by introducing the microorganism in the substrate solution and oxygenating the substrate solution by letting air in a finely divided state bubble through the substrate solution. The degree of fine division of the air and, thus, the oxygen transfer efficiency are determined substantially by the intensity of agitation in the bioreactor. An upper limit for the intensity of agitation is set by the physiology of the organism. All organisms, and in particular those producing mycelium, are sensitive to the shear stresses which appear upon intense agitation. Another very common technique is to place the microorganism on an inert carrier, such as crushed slag, macadam, pumice etc., and have the substrate solution flush a bed of the microorganism coated on the carrier while aerating the bed. One example of this are the biological beds which are used for treating wastewater. In these prior art methods, the supply of substrate solution to the microorganism is no major problem but can easily be performed in a quick and efficient way. On the other hand, it is difficult to achieve an optimally efficient addition of oxygen to the microorganism. In fact, in order that the microorganism should be able to use the oxygen, this must diffuse through the liquid phase of substrate solution surrounding the microorganism. Such diffusion through the liquid phase normally is very slow and is the stage which determines the velocity of the entire process. Therefore, several attempts have been made to facilitate and accelerate the diffusion, for instance by agitation, finely dividing the air, dividing the substrate solution into fine droplets, etc. Although these measures give a certain improvement, they require relatively large amounts of energy.

For instance, the costs of aeration often is, next to costs for raw material and equipment, the largest cost in industrial microbiological processes. Moreover, the fine division effected to increase the liquid-gas contact surface generally is not very efficient.

Thus, even if the substrate solution is divided into fine droplets, each droplet has a substantial mass or bulk into which the air can diffuse entirely only after a relatively long period of time.

It appears from the above that the oxygen-transferring capacity of the system which is decisive of the velocity of aerobic microbiological processes is not optimal in systems hitherto used, which is a serious drawback, among other things because it sets an upper limit to final cell concentration or productivity in the substrate. In microbiological processes, it is desirable almost without exception to have this concentration or productivity as high as possible.

It is therefore a primary object of the invention to achieve an aerobic microbiological method in which these drawbacks are obviated and which ensures an efficient supply of both substrate solution and air in a manner which does not adversely affect the microorganism.

It is a further object of the invention to achieve an aerobic microbiological method in which the microorganism is efficiently immobilized on a carrier.

Yet another object of the invention is to achieve an aerobic microbiological method in which the growth of the microorganism is maintained at an optimal level during the entire process.

These and other objects and advantages of the invention are achieved by an aerobic microbiological method in which a microorganism is immobilized in a fluid-permeable matrix and supplied with a substrate solution and an oxygen-containing gas, characterized in that the microorganism is immobilized in a three-dimensional fibre matrix, that the substrate solution and the oxygen-containing gas are supplied to and leave the matrix as separate flows mutually at an angle of at least 10°, whereby the substrate solution and the oxygen-containing gas are brought into cross-flow contact with each other and with the microorganism within the matrix.

Further features of the invention will appear from the subclaims.

The angle between the flow of substrate solution and the flow of oxygen-containing gas preferably is at least 45° and most preferably about 90°.

In this context, it should be noted that it is per se known to use a matrix of for instance mineral wool as a biological bed in the treatment of wastewater. This appears for instance from SE patent application 7308380—0 which relates to a device for biochemical degradation of desludged wastewater. The device contains one or more porous filters which may consist of mineral wool, and the wastewater to be treated is allowed to trickle down through the filters while air is flowing both underneath and above as well as through the filters. Thus, in this case it is not a matter of an adjusted cross-flow and, above all, not of an adjusted cross-flow within the filters, but the contact between air and liquid takes place substantially outside the filters where the liquid falls as droplets through the air flow above and beneath the filters. This makes the known device suffer precisely from the shortcomings of the art of poor oxygen transfer capacity as mentioned above.

Another example of the prior art is disclosed in SE patent application 7502057—8 which relates to a method and an apparatus for purifying water. In this patent application, oxygenated or aerated water is conducted through a filter bed of e.g. mineral wool in the form of one or more layers. This method suffers to an even greater extent from the above-mentioned drawbacks, and the advantageous cross-flow within the fibre matrix as described in this application is not disclosed.

Yet another example of the prior art is disclosed in NO patent 147,639 which relates to a biological contact filter having several superposed beds with perforated bottoms. The uppermost bed may consist of mineral wool. The filter is aerated by supplying air from underneath, i.e. the filter operates by countercurrent contact between liquid and air.

US patent specification 4,165,281 finally discloses wastewater treatment where active sludge is fixed on fibre mats of synthetic material such as nylon, PVC and the like. The mats are disposed suspended in flowing and aerated wastewater, i.e. there is no cross-flow of air and wastewater within the mats.

In connection with the present invention, it has been found that if a liquid is conducted through a three-dimensional fibre matrix the fibres of which are wetted by the liquid, and a gas is at the same time pressed through the matrix in cross-flow with respect to the liquid flow, the gas/liquid contact will become intense. The liquid flows as a thin film along certain fibres. Droplets flowing along other fibres are subjected to agitation at the points of intersection of the fibres. Droplets growing large and falling off one fibre will be broken up as they impinge on another fibre. This results in an intense physical agitation of the liquid. In conventional gas/liquid contact devices, the oxygen transfer capacity or matter transport of the dissolved gas from the saturated liquid surface to the unsaturated inner portions of the liquid is, as mentioned above, the velocity-determining stage. By the intense agitation and fine division of the liquid taking place in accordance with the present invention, this limitation is substantially eliminated, which makes the apparatus according to the invention considerably more efficient per unit of volume than conventional devices.

In addition to the improved transfer of oxygen obtained by the cross-flow of air and substrate solution within the fibre matrix according to the present invention, the invention in its preferred embodiments offers additional advantages.

Thus, the invention provides an improved method for immobilizing or fixing the microorganisms in the fibre matrix in that the fibre matrix after introduction of the preferably aggregated microorganisms is compressed so that the microorganisms are fixed within the matrix.

The distribution of the microorganisms in the vertical direction of the matrix may be further improved by precompressing the matrix before introducing the microorganism. Normally, the precompression is then made greater at one end of the matrix and successively decreases towards the opposite end of the matrix. Such an unsymmetrical or "conical" precompression entails that the microorganism when introduced will be more evenly and uniformly distributed in the matrix. This is of particular importance when grafting clusters of organisms as these aggregates in a normal method of production on a larger scale will have a wide size distribution. When the introduction of the microorganism is terminated, the matrix can be compressed, as described above, this subsequent compression being suitably so effected that the total compression of the matrix becomes substantially uniform, i.e. the matrix is subsequently compressed most at the insertion end and least at the opposite end. Alternatively, the matrix may be decompressed, i.e. the precompressed end is allowed to expand.

A further essential advantage of a preferred embodiment of the invention is to allow the matrix to expand in dependence upon the growth of the microorganisms during the process. Preferably, the expansion of the matrix is so controlled that the growth of the microorganism is optimal all the time. One way of controlling this expansion or increase in volume of the matrix is maintaining an essentially constant liquid flow through the matrix, i.e. the volume of the matrix is increased continuously such that the liquid flow is maintained substantially constant all the time. Another way of controlling the expansion is to keep the pressure drop in the air flow through the matrix constant, i.e. to continuously increase the volume of the matrix during the process such that the pressure drop will be maintained substantially constant all the time. Yet another way of controlling the expansion is to ensure that the liquid or air flow is maintained correlated to the cell mass existing at each point of time.

An important technical and economic factor is the pressure drop of the gas flow through the matrix. In a matrix of glass wool with a density of 23 kg/m³ which is a normal value for a glass-wool slab for building purposes, the volume of the matrix consists of about 1% glass and 99% voids. Also relatively large amounts of liquid can pass through such a matrix without its voids being filled by more than a few per cent, which means that the gas pressure drop is low also with a simultaneous flow of liquid.

The fibre matrix used in the method according to the invention may in principle consist of any type of organic or inorganic fibre material, such as polymer fibres, for instance fibres of polyvinyl chloride, polyethylene, polypropylene, polyacrylonitrile, polyvinyl alcohol, cellulose, cellulose derivatives etc, or inorganic fibres, for instance fibres of metals, metal oxides, glass, ceramic material etc.

According to the invention, it is however preferred more particularly to use a fibre matrix of mineral wool, such as glass wool. Such fibre matrices have all the properties which are required in a matrix according to the present invention, such as inertness, low resistance to gas and liquid flow, good dimensional stability etc, and can also be manufactured at a low cost. Since mineral wool, such as glass wool and rock wool, thus is a superior matrix material, the invention will be described hereinafter with reference to mineral wool.

The fibres of the matrix have a diameter of about 1—500 µm, preferably about 1—100 µm and most preferably about 1—20 µm. In order that the fibres should be sufficiently wetted, they should be hydrophilic in themselves or be treated so as to become hydrophilic.

As mentioned above, the matrix according to the present invention is three-dimensional, which means that it has an extent in each of three planes at right angles to each other of at least 10 times the fibre diameter. In order to increase the self-supporting capacity of the three-dimensional fibre matrix, the fibres of the matrix at their points of intersection may be linked together by chemical or mechanical bonds. One example of chemical bonding is interconnecting the fibres at their points of intersection by means of polymer binders, for instance of the phenolic resin type. Another example of bonding is fusing the fibres at their points of intersection by heat or by means of a solvent. An example of mechanical bonding is needling the fibre material. A three-dimensional matrix thus bonded is substantially self-supporting, which means that a particular equipment for encapsulating the matrix is normally not required. It may however be desirable or suitable in some cases to provide the matrix element with external support means which may be designed in a simple and inexpensive way as gas-permeable walls of e.g. wire netting or perforated metal sheets.

In its simplest embodiment, the matrix consists of a homogeneous fibre body, i.e. of fibres having substantially the same size and properties. In order to counteract penetration of liquid from the downwardly flowing liquid at the vertical boundary walls of the matrix, the outer vertical surfaces of the matrix may be made hydrophobic by treating the fibres in these outer surfaces with hydrophobating oils, waxes or polymers in a per se known way. In these outer layers, the fibres are thus not wetted by the liquid, for which reason the resistance to liquid penetration is high while the gas pressure drop is maintained low. This means that the outer layers serve as an outer boundary to the inner wetted layers of the matrix and allow the gas to pass therethrough whereas not the liquid.

Further alternative embodiments of the matrix according to the invention include multi-layer matrices in which the matrix body is composed of a plurality of different fibre layers which are distinct or continuously merging into each other and which differ by their fibre diameter, distribution of fibre diameter, fibre length, density etc. These fibre layers are suitably arranged in parallel beside each other or concentrically around each other in the direction of flow of the liquid. In the case of distinct fibre layers, the layers may either engage each other directly or be separated by intermediate layers which are preferably hydrophobic.

In order to facilitate the understanding of the invention, it will be described in greater detail hereinbelow with reference to the accompanying drawings. In the drawings, Fig. 1 is a schematic view of the cross-flow technique according to the invention; Fig. 2 illustrates the compression of the fibre matrix in connection with the introduction of the microorganism; Fig. 3 illustrates the oblique precompression of the matrix; and Fig. 4 illustrates how the volume increase of the matrix is adjusted during the process as a function of the air pressure over the matrix.

When carrying out the method according to the invention, the microorganism is first introduced in the fibre matrix, i.e. the matrix is "grafted" with the microorganism. This is effected in such a way that the microorganism as such or suspended in a suitable liquid is supplied to the fibre matrix and retained therein by the filter action of the matrix.

Both the initial retention and the subsequent retention of any newly produced cells may, in addition to a pure "screening action" (mechanical retention), also consist of other kinds of interaction between the microorganism and the fibre, such as electric forces of attraction, chemical bonds, phenomena of adhesion pertaining to surface chemistry or physics and ongrowth. The method is especially well suited for any immobilization technique which does not strongly inhibit the supply of oxygen to the organism.

It is not certain that all microorganisms will immediately adhere to and be retained in the matrix. If so, the liquid is suitably recycled with the suspended microorganism and reintroduced in the matrix. This recirculation is repeated until the desired degree of grafting of the matrix with the microorganism has been obtained.

The liquid in which the microorganism is suspended when grafting is performed may consist either of a complete substrate solution which contains all the nutrients essential to the growth of the microorganism, or of an incomplete substrate solution which lacks one or more nutrients, whereby the desired metabolic activity of the microorganism is maintained while its growth is counteracted.

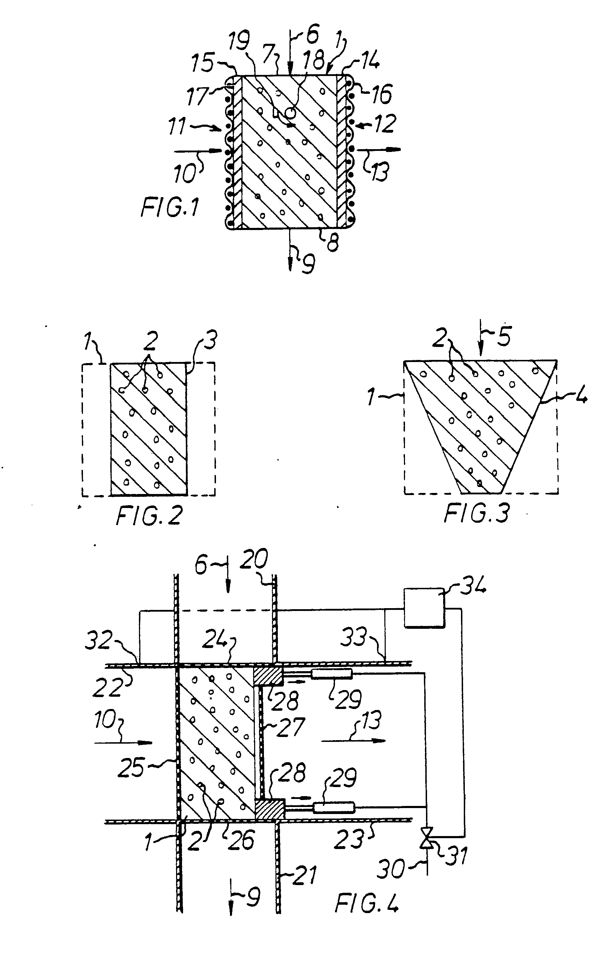

In order to improve the retention of the microorganism in the fibre matrix, it may be suitable first to aggregate the microorganism in a per se known manner and then introduce the microorganism in the aggregated state in the matrix. In order further to enhance the retention of the microorganism in the matrix, a preferred embodiment of the invention provides for compressing the matrix after the microorganism has been introduced. By this subsequent compression, the microorganism will be fixed efficiently in the matrix and prevented from leaving it. Such subsequent compression is schematically illustrated in Fig. 2 where the initial extent of the matrix 1, as intimated by dashed lines, has been compressed after introduction of the microorganism 2, indicated by dots, to the final volume indicated by full lines.

In order further to facilitate the introduction of the microorganism in the matrix and especially to obtain a uniform distribution of the microorganism, the matrix according to a second preferred embodiment of the invention is precompressed before the microorganism is introduced. Such precompression is schematically indicated in Fig. 3 and is so effected that the matrix 1 is compressed unsymmetrically, so that one end of the matrix (the lower end in Fig. 3) is strongly compressed while the compression successively decreases towards the opposite end of the matrix. A precompressed matrix will thus be obtained having a conical cross-section configuration as shown by full lines in Fig. 3. As shown by an arrow 5, the microorganism is thereafter introduced at the uncompressed end of the matrix and penetrates into the matrix, a substantially uniform distribution of the microorganism taking place in the matrix, as intimated by the dots 2, in that the matrix, as counted from the end of introduction, has a progressively increasing compression.

A matrix 1 with microorganisms 2 is schematically shown in Fig. 1. For carrying out the microbiological method according to the invention, a substrate solution 6 is supplied to one of the side faces 7 of the matrix and, after passing through the matrix, the substrate solution leaves the matrix at the opposite side face 8, as shown by the arrow 9. In order to bring about the favourable cross-flow aimed at according to the invention of substrate solution and air within the matrix 1, air 10 is supplied to one side face 11 of the matrix. In the illustrated embodiment in Fig. 1, the side face 11 is substantially at right angles to the side face 7 to which the substrate solution 6 is supplied, i.e. the relative angle of the flow 6 of substrate solution to the air flow 10 is 90°. The air 10 is brought into an intense contact with the substrate solution 6 and the microorganism 2 within the matrix 1 under cross-flow conditions and thereafter leaves the matrix at a side face 12 opposite the side face 11, as shown by the arrow 13. It should be pointed out that the method shown in Fig. 1 is illustrated highly schematically, the flows of substrate solution and air in the method according to the invention forming distinct flows which are separated from each other, both before and after passing through the matrix 1. This may be readily obtained by feed and discharge conduits or channels (not shown). Essential is that the cross-flow contact between substrate solution and air takes place within the very matrix with the microorganism 2.

When using a homogeneous hydrophilic matrix 1, as in Fig. 1, there is a risk of liquid penetration at the outer surfaces of the matrix, especially on the "leeward side" (to the right in Fig. 1) of the air flow. To counteract this, the matrix may be provided with hydrophobic outer layers 14, 15, as shown in Fig. 1. As previously mentioned, these hydrophobic outer layers are gas-permeabile but substantially liquid-impermeable. In the cases where the hydrophobic outer layers have sufficient stiffness, they may also serve as outer support means. The wire netting 16, 17 shown in Fig. 1 may then be dispensed with.

The substrate solution 6 migrating down through the matrix 1 will be urged by the pressure from the through-flowing air 10, towards one side of the matrix (the right-hand side in Fig. 1). This means that the substrate solution has a tendency not to leave the matrix exactly underneath its point of ingress in the matrix but at a point slightly offset in the lateral direction. In order to counteract such lateral offsetting of the substrate solution 9 leaving the matrix, this may be slightly inclined such that the substrate solution will have a tendency to migrate towards the opposite outer face of the matrix, this tendency being equal but contrary to the tendency towards said lateral offsetting. Further, a modified fibre orientation may counteract such lateral offsetting. The very inclination of the matrix may either be made permanent or be carried out automatically and self-adjustingly by mounting the matrix on a horizontal shaft 18 extending at right angles to the air flow 10 above the centre of gravity of the matrix. The pressure exerted by the air flow 10 against the matrix 1 will produce a torque which tends to turn the matrix about the shaft 18 in the direction of the arrow 19. With a shaft 18 suitably arranged, the then obtained inclination of the matrix 1 may be made exactly as great as to counteract the lateral offsetting of the substrate solution 9 leaving the matrix.

Another important and preferred aspect of the present invention is to increase the volume of the matrix in dependence upon the growth of the microorganism during the microbiological process. A currently preferred solution of how to achieve such a controlled volume increase is schematically illustrated in Fig. 4. In this case, the matrix 1 with the microorganism 2 is arranged such that the substrate solution 6 is supplied from above through a supply conduit 20, passes through the matrix and leaves it at its lower end as a flow of spent substrate solution 9 through the discharge conduit 21. The air 10 is supplied through an air-supply conduit 22 substantially at right angles to the conduit 20 and penetrates into the matrix 1 where cross-flow contact takes place between the substrate solution 6 and the air flow 10, whereupon the air leaves the matrix at the opposite side face as an air flow 13 through a conduit 23. The matrix 1 is kept in place in the apparatus by means of nets or perforated metal sheets 24, 25, 26 in the conduits 20, 22 and 21, respectively. Moreover, there is a positioning net or grating 27 mounted in the conduit 23, said grating being stretched between holders 28 which can be moved back and forth in the horizontal direction by means of hydraulic cylinders 29 actuated by hydraulic fluid from a conduit 30 with a control valve 31.

Further, there are pressure-sensing probes 32 and 33 in the air-supply conduit 22 and the air-discharge conduit 23, respectively, the probes 32 and 33 being connected to a control means 34 actuating the valve 31 in dependence upon the pressure difference sensed by the probes 32 and 33. The apparatus operates as follows:

Substrate solution 6 and air 10 pass in a cross-flow through the fibre matrix 1 with the microorganisms 2. The perforated positioning wall 27 then is in the position shown in Fig. 4. As the microbiological process proceeds, the microorganisms grow and multiply, which means that an ever increasing proportion of the volume of the fibre matrix 1 is occupied by the microorganism 2. This, in turn, means that the fibre matrix 1 with the microorganism 2 offers increased resistance to the air flow 10, this resistance being sensed by the pressure-sensitive probes 32 and 33 which emit a signal to the control means 34. The control means 34 then emits a control signal to the valve 31 which opens to the hydraulic fluid such that the hydraulic cylinders 29 can retract the means 28 and, thus, the perforated positioning grating 27. The fibre matrix 1 will then have an increased volume at its disposal, i.e. it is free to expand to the right in Fig. 4. This means that the matrix no longer offers an equally great resistance to the air flow, i.e. the pressure drop of the air flow across the matrix has decreased. Suitably, the control means 34 is so set that the pressure drop of the air flow through the matrix 1 is maintained constant. Consequently, this means that the adjustable positioning wall 27 during the microbiological process is successively moved to the right in Fig. 4.

In the foregoing, the invention has been described with reference to preferred embodiments only, but it is evident that it is not restricted thereto, its full scope being determined by the accompanying claims.

**Claims**

1. An aerobic microbiological method in which a microorganism (2) is immobilized in a fluid-permeable matrix (1) and supplied with a substrate solution (6) and an oxygen-containing gas (10), characterized in that the microorganism (2) is immobilized in a three-dimensional fibre matrix (1), that the substrate solution (6) and the oxygen-containing gas (10) are supplied to and leave the matrix (1) as separate flows (10, 13 and 6, 9 respectively), mutually at an angle of at least 10°, whereby the substrate solution (6) and the oxygen-containing gas (10) are brought into cross-flow contact with each other and with the microorganism (2) within the matrix (1).

2. Method as claimed in claim 1, characterized in that the microorganism (2) is immobilized in a three-dimensional fibre matrix (1) which essentially consists of mineral wool.

3. Method as claimed in claim 1 or 2, characterized in that the microorganism (2) is immobilized by introducing a predetermined amount of the microorganism in the matrix (1) and thereafter compressing the matrix.

4. Method as claimed in any one of claims 1—3, characterized in that the microorganism is introduced at one end of a precompressed matrix.

5. Method as claimed in any one of claims 1—4, characterized in that the oxygen-containing gas (10) supplied is air.

6. Method as claimed in any one of claims 1—5, characterized in that the substrate solution (6) is supplied at the upper end (7) of the matrix and by gravity flows substantially vertically through the matrix (1).

7. Method as claimed in any one of claims 1—6, characterized in that it is performed at a temperature of 0—100°C.

8. Method as claimed in any one of claims 1—7, characterized in that the volume of the matrix is increased in dependence upon the growth of the microorganism.

9. Method as claimed in any one of the preceding claims, characterized in that the matrix (1) is vertically inclined such that its lower end (8) is offset in the direction of flow (13) of the gas with respect to the upper end (7) of the matrix.

10. Method as claimed in any one of the preceding claims, characterized in that the oxygen-containing gas (13) leaves the matrix (1) at a surface which has been made hydrophobic (14).

**Patentansprüche**

1. Aerobisches mikrobiologisches Verfahren, bei welchem ein Mikroorganismus (2) in einer fluiddurchlässigen Matrize (1) immobilisiert und mit einer Substratlösung (6) und einem Sauerstoff enthaltenden Gas (10) versorgt wird, dadurch gekennzeichnet, dass der Mikroorganismus (2) in einer dreidimensional Fasermatritze (1) immobilisiert wird, und dass die Substratlösung (6) und das Sauerstoff enthaltende Gas (10) der Matrize (1) als separate Strömungen (10, 13 bzw, 6, 9) zugeführt werden und die Matrize auch separat wieder verlassen, und zwar in einem gegenseitigen Winkel von zumindest 10°, wodurch die Substratlösung (6) und das Sauerstoff enthaltende Gas (10) in Querströmungsberührung mit einander und mit dem Mikroorganismus (2) in der Matrize (1) gebracht werden.

2. Verfahren nach Anspruch 1, dadurch gekennzeichnet, dass der Mikroorganismus (2) in einer dreidimensionalen Fasermatritze (1) immobilisiert wird, die hauptsächlich aus Mineralwolle besteht.

3. Verfahren nach Anspruch 1 oder 2, dadurch gekennzeichnet, dass der Mikroorganismus (2) dadurch immobilisiert wird, dass eine vorbestimmte Menge des Mikroorganismus in die Matrize (1) eingebracht wird, wonach die Matrize zusammengepresst wird.

4. Verfahren nach einem der Ansprüche 1—3, dadurch gekennzeichnet, dass der Mikroorganismus am einen Ende einer im voraus zusammengepressten Matrize eingebracht wird.

5. Verfahren nach einem der Ansprüche 1—4, dadurch gekennzeichnet, dass das zugeführte, Sauerstoff enthaltende Gas (10) Luft ist.

6. Verfahren nach einem der Ansprüche 1—5, dadurch gekennzeichnet, dass die Substratlösung (6) am oberen Ende (7) der Matrize zugeführt wird und unter dem Einfluss der Schwerkraft hauptsächlich senkrecht durch die Matrize (1) strömt.

7. Verfahren nach einem der Ansprüche 1—6, dadurch gekennzeichnet, dass das Verfahren bei einer Temperatur von 0—100°C ausgeführt wird.

8. Verfahren nach einem der Ansprüche 1—7, dadurch gekennzeichnet, dass das Volumen der Matrize entsprechend dem Zuwachs des Mikroorganismus erhöht wird.

9. Verfahren nach einem der vorhergehenden, Ansprüche, dadurch gekennzeichnet, dass die Matrize (1) derart schräggestellt wird, dass ihr unteres Ende (8) im Verhältnis zu ihrem oberen Ende (7) in der Strömungsrichtung (13) des Gases versetzt ist.

10. Verfahren nach einem der vorhergehenden Ansprüche, dadurch gekennzeichnet, dass das Sauerstoff enthaltende Gas (13) die Matrize (1) an einer hydrophob (14) gemachten Oberfläche verlässt.

## Revendications

1. Une méthode microbiologique aérobie selon laquelle un micro-organisme (2) est immobilisé dans une matrice perméable aux fluides (1) qui est alimentée par une solution de substrat (6) et un gaz contenant de l'oxygène (10), caractérisée en ce que le micro-organisme (2) est immobilisé dans une matrice de fibres tridimensionnelle (1), et en ce que la solution de substrat (6) et le gaz contenant de l'oxygène (10) sont alimentés à la matrice (1) et quittent celle-ci sous forme de courants séparés (10, 13 et 6, 9 respectivement), faisant mutuellement un angle d'au moins 10°, de sorte que la solution de substrat (6) et le gaz contenant de l'oxygène (10) sont mis en contact en écoulement croisé l'un avec l'autre et avec le micro-organisme (2) à l'intérieur de la matrice (1).

2. Méthode selon la revendication 1, caractérisée en ce que le micro-organisme (2) est immobilisé dans une matrice de fibres tridimensionnelle (1) qui consiste essentiellement en laine minérale.

3. Méthode selon la revendication 1 ou 2, caractérisée en ce que le micro-organisme (2) est immobilisé par introduction d'une quantité prédéterminée du micro-organisme dans la matrice (1) et ensuite par compression de la matrice.

4. Méthode selon l'une quelconque des revendications 1—3, caractérisée en ce que le micro-organisme est introduit à une extrémité d'une matrice précomprimée.

5. Méthode selon l'une quelconque des revendications 1—4, caractérisée en ce que le gaz contenant de l'oxygéne (10) alimenté est de l'air.

6. Méthode selon l'une quelconque des revendications 1—5, caractérisée en ce que la solution de substrat (6) est alimentée à l'extrémité supérieure (7) de la matrice et s'écoule par gravité substanteillement verticalement à travers la matrice (1).

7. Méthode selon l'une quelconque des revendications 1—6, caractérisée en ce qu'elle est conduite à une température de 0—100°C.

8. Méthode selon l'une quelconque des revendications 1—7, caractérisée en ce que le volume de la matrice est accru en fonction de la croissance du micro-organisme.

9. Méthode selon l'une quelconque des revendications précédentes, caractérisée en ce que le matrice (1) est inclinée verticalement de sorte que son extrémité inférieure (8) est décalée dans la direction d'écoulement (13) du gaz par rapport à l'extrémité supérieure (7) de la matrice.

10. Méthode selon l'une quelconque des revendications précédentes, caractérisée en ce que le gaz contenant de l'oxygène (13) quitte la matrice (1) par une surface qui a été rendue hydrophile (14).

FIG.1

FIG. 2

FIG.3

FIG. 4

1